# EUROPEAN PATENT SPECIFICATION

(11) **EP 0 418 280 B1**
(45) Date of publication and mention of the grant of the patent: **09.02.1994**
(21) Application number: 89906260.8
(22) Date of filing: 24.04.1989
(51) Int. Cl.: C07C 69/157, C07C 67/24

(54) **METHOD OF PREPARING ARYL ESTERS OF ACETIC ACID**
HERSTELLUNGSVERFAHREN VON ESSIGSÄURE ARYLESTERN
PROCEDE DE PREPARATION D'ESTERS D'ARYLE D'ACIDE ACETIQUE

(30) Priority: 02.05.1988 US 189085
(43) Date of publication of application: 27.03.1991
(73) Proprietor: EASTMAN CHEMICAL COMPANY, Kingsport, TN 37660 (US)
(72) Inventor: LARKINS, Thomas, Hassell, Jr., Kingsport, TN 37664 (US)
(74) Representative: Behrens, Dieter, Dr.-Ing.
(86) International application number: US8901681
(87) International publication number: WO8910913

(56) References cited:
- DE-A- 1 493 211
- FR-A- 0 794 715

## Description

The invention relates to a method of reacting an aryl ether with acetic acid and acetic anhydride to form an aryl ester of acetic acid.

Aryl esters of acetic acid are highly useful commercial compounds. Such compounds are used as solvents, perfumes and intermediates in reactions wherein other functional groups are introduced into the aromatic ring. In addition, these compounds can be employed in the formation of polymers.

It has been known in the past to form alkyl esters by cleavage of ether compounds. In particular, it is well known to cleave the ether linkage by addition of an organic acid in the presence of a strong acid. For example in French patent 794 715 isopropyl acetate is obtained by reacting diisopropyl ether with acetic acid in the presence of sulfuric acid. There is still a need, however, for a method of producing aryl esters cheaply and conveniently.

According to the present invention, aryl esters of acetic acid are prepared by a method comprising reacting an ether having the formula [R-O]ₙ-R', wherein R is a substituted or unsubstituted alkyl group, R' is a substituted or unsubstituted aryl group, with an acetylating agent selected from the group consisting of acetic acid and acetic acid anhydride in the presence of at least stoichiometric amounts of an alkali metal iodide salt, to form an aryl ester of acetic acid, an alkali metal salt of acetic acid, and an alkyl iodide.

In the method of the present invention, the alkali metal iodide and the acetylating agent should be present in at least about a molar equivalent amount per molar equivalent of the aryl ether group. It is particularly preferred that the aryl ether group, the acetylating agent, and the alkali metal iodide salt are present in about stoichiometric amounts.

The R group in the ether can be alkyl containing from about 1 to 15 carbons. Particularly preferred are the lower alkyl groups, e.g., of about 1 to 5 carbon atoms. R may also be substituted with groups such as hydroxy, carboxyl and amino groups. Illustrative of suitable R groups are methyl, ethyl, propyl, isopropyl, butyl, pentyl, hexyl, octyl, nonyl, decyl, dodecyl, and the like.

The R' group can contain at least 6 carbon atoms and up to about 15 carbon atoms. Exemplary of suitable R' groups are phenyl, methylphenyl, ethylphenyl, diphenyl, benzyl, methylbenzyl, naphthyl, and the like. Additionally, the aryl groups can be substituted with groups such as hydroxy, amino, carboxyl and alkoxy.

Since n can be 1, 2, 3 or 4 the ethers suitable for use in the present invention can include aryl monoalkyl ethers such as hydroquinone monoethyl ether, hydroquinone monoisopropyl ether, resorcinol monomethyl ether, resorcinol monoethyl ether, guaicol, methoxybenzoic acid, ethoxybenzoic acid, butoxybenzoic acid, methoxyphenylamine, ethoxyphenyl amine, or dialkyl ethers such as hydroquinone dimethyl ether hydroquinone diethyl ether, hydroquinone diisopropyl ether, 3, 4-dimethoxybenzoic acid, and 3, 4-dimethoxy-5-hydroxy-benzoic acid.

In the present invention, an acetylating agent is used to react with the ether in order to form the aryl ester of acetic acid. The acetylating agent used can be either acetic acid or acetic acid anhydride or a combination of both acetic acid and acetic anhydride.

To form the aryl ester of acetic acid the aryl ether described above is reacted with acetic acid or acetic anhydride in the presence of an alkali metal iodide salt. Preferably, the alkali metal of the iodide salt is either sodium or lithium, although other alkali metals such as magnesium and potassium may be employed. The sodium iodide or lithium iodide is preferably added to the reaction in anhydrous form.

In the reaction of the present invention an aryl ester of acetic acid is produced along with an alkali metal salt of acetic acid and an alkyl iodide. The production of the aryl ester is accomplished in this reaction via the replacement of the ether groups on the aromatic ring of the aryl ether by acetoxy groups. For example, when hydroquinone dimethyl ether is the aryl ether, an acetoxy group replaces the ether linkage, and the resulting product is acetoxy anisole. The acetoxy groups can subsequently be removed by hydrolysis or other chemical reactions, such as polymerization reactions.

Where the aromatic ether employed in the present invention is a methyl or dimethyl aryl ether, the alkyl iodide formed will be methyl iodide. Methyl iodide can be processed in several ways to allow for iodine recovery. For instance, there are many industrial processes which require methyl iodide as a reactant. Further, methyl iodide can be catalytically oxidized in air to form elemental iodine which can be recovered and recycled for use in iodination reactions such as the iodination reaction that prepares iodoaromatic compounds.

Where the alkali metal iodide salt used in the present reaction is sodium or lithium iodide, the resulting alkali metal salt of acetic acid produced will be sodium acetate or lithium acetate, respectively. The sodium or lithium acetate thus produced can be recovered and can be used in the preparation of aromatic ethers described below.

The present invention is preferably carried out by reacting in an autoclave a mixture of a methyl, dimethyl, or trimethyl aryl ether, anhydrous sodium or lithium iodide, and acetic acid. The sodium or lithium iodide should be present in at least stoichiometric quantities. Before heating, the autoclave is closed and about 689.5 kPa (100 psig) of nitrogen gas is added at room temperature. The autoclave should then be heated so that the reaction takes place at a temperature between about 130°C to about 225°C. Preferably, the reaction is conducted at a temperature of about 160°C to about 185°C. The reaction mixture should be heated for about 1-3 hours, after which the autoclave is cooled for about one hour, opened, and the pressure vented. At this point, the aryl ester of acetic acid will have formed, and the sodium or lithium iodide will have been converted to methyl iodide and sodium or lithium acetate. The aryl ester can be separated out from unreacted methyl or dimethyl aryl ether and recovered, and the methyl iodide and metallic acetate salt can be recovered and recycled.

The following examples are presented as illustrative of the present invention and should not be construed as limiting the invention in any way.

### Example 1

A 300 mL Hastelloy C rocking autoclave was loaded with 10.0 g (0.072 M) hydroquinone dimethyl ether, 20.0 g (0.15 M) anhydrous lithium iodide, and 200 mL acetic acid. The autoclave was closed and 689.5 kPa (100 psig) nitrogen gas was added at room temperature. The autoclave was heated to 175°C over a 45 minute period and held at 175°C for two hours. The autoclave was cooled over a one hour period. The pressure was vented and the autoclave was opened. As determined by gas chromatographic methods of analysis, the liquid product contained 0.018 M hydroquinone dimethyl ether, 0.024 M hydroquinone monomethyl ether, 0.013 M acetoxy anisole, 0.016 M hydroquinone monoacetate, and 0.002 M hydroquinone diacetate. The lithium iodide was converted to methyl iodide and lithium acetate.

### Example 2

As in Example 1, 10.0 g (0.072 M) hydroquinone dimethyl ether, 20.0 g (0.133 M) anhydrous sodium iodide, and 200 mL acetic acid were heated two hours at 175°C. The liquid product contained 0.040 M hydroquinone dimethyl ether, 0.017 M hydroquinone monomethyl ether, 0.011 M acetoxy anisole, 0.006 M hydroquinone monoacetate, and a trace of hydroquinone diacetate. The sodium iodide was converted to methyl iodide and sodium acetate.

### Example 3

As in Example 1, 10.0 g (0.072 M) hydroquinone dimethyl ether, 30.0 g (0.20 M) anhydrous sodium iodide, 15 mL (0.15 M) acetic anhydride and 185 mL acetic acid were heated two hours at 185°C. The liquid product contained 0.017 M hydroquinone dimethyl ether, 0.038 M acetoxy anisole, 0.010 M hydroquinone diacetate and a trace of hydroquinone monoacetate.

### Example 4

As in Example 1, 10.0 g (0.072 M) hydroquinone dimethyl ether, 30.0 g (0.22 M) anhydrous lithium iodide, 15 mL (0.15 M) acetic anhydride, and 185 mL acetic acid were heated two hours at 205°C. The liquid product contained 0.005 M acetoxy anisole, 0.063 M hydroquinone diacetate, and traces of hydroquinone dimethyl ether and hydroquinone monoacetate.

### Example 5

As in Example 1, 10.0 g (0.072 M) hydroquinone dimethyl ether, 50 g (0.33 M) anhydrous sodium iodide, 15 mL (0.15 M) acetic anhydride, and 185 mL acetic acid were heated two hours at 215°C. The liquid product contained 0.005 M acetoxy anisole, 0.004 M hydroquinone monoacetate, and 0.063 M hydroquinone diacetate.

### Example 6

As in Example 1, 10.0 g (0.072 M) hydroquinone dimethyl ether, 38.6 g (0.29 M) anhydrous lithium iodide, 15 mL (0.15 M) acetic anhydride, and 100 mL acetic acid were heated one hour at 190°C. The liquid product contained 0.001 M hydroquinone monoacetate, 0.073 M hydroquinone diacetate, and a trace of hydroquinone dimethyl ether.

## Claims

1. A method of preparing an aryl ester of acetic acid comprising reacting an ether having the formula [R-O]ₙ-R', wherein R is a substituted or unsubstituted alkyl group, R' is a substituted or unsubstituted aryl group, and n is 1, 2, 3 or 4 with an acetylating agent selected from the group consisting of acetic acid and acetic acid anhydride in the presence of at least stoichiometric amounts of an alkali metal iodide salt.

2. The method of Claim 1, wherein the ether, the acetylating agent and the alkali metal iodide salt are present in about stoichiometric amounts.

3. The method of Claim 1, wherein the reaction is conducted at a temperature of about 130°C to about 225°C.

4. The method of Claim 3, wherein the reaction is conducted at a temperature of about 160°C to about 185°C.

5. The method of Claim 1, wherein said ether comprises a methyl aryl ether.

6. The method of Claim 1, wherein said ether comprises a dimethyl aryl ether.

7. The method of Claim 1, wherein the alkali metal of the iodide salt is selected from the group consisting of lithium and sodium.

8. The method of Claim 7, wherein the alkali metal is lithium.

9. The method of Claim 7 wherein the alkali metal is sodium.

## Patentansprüche

1. Verfahren zur Herstellung eines Arylesters der Essigsäure, bei dem man einen Ether der Formel [R-O]ₙ-R', worin R eine substituierte oder unsubstituierte Alkylgruppe ist, R' eine substituierte oder unsubstituierte Arylgruppe darstellt und n gleich 1, 2, 3 oder 4 ist, mit einem Acetylierungsmittel umsetzt, das ausgewählt ist aus der Gruppe bestehend aus Essigsäure und Essigsäureanhydrid, in Gegenwart von mindestens stoichiometrischen Mengen eines Alkalimetalliodidsalzes.

2. Verfahren nach Anspruch 1, bei dem der Ether, das Acetylierungsmittel und das Alkalimetalliodidsalz in etwa stoichiometrischen Mengen vorliegen.

3. Verfahren nach Anspruch 1, bei dem die Reaktion bei einer Temperatur von etwa 130°C bis etwa 225°C durchgeführt wird.

4. Verfahren nach Anspruch 3, bei dem die Reaktion bei einer Temperatur von etwa 160°C bis etwa 185°C durchgeführt wird.

5. Verfahren nach Anspruch 1, bei dem der Ether ein Methylarylether ist.

6. Verfahren nach Anspruch 1, bei dem der Ether ein Dimethylarylether ist.

7. Verfahren nach Anspruch 1, bei dem das Alkalimetall des Iodidsalzes ausgewählt ist aus der Gruppe bestehend aus Lithium und Natrium.

8. Verfahren nach Anspruch 7, bei dem das Alkalimetall Lithium ist.

9. Verfahren nach Anspruch 7, bei dem das Alkalimetall Natrium ist.

## Revendications

1. Méthode de préparation d'un ester d'aryle d'acide acétique comprenant la mise en réaction d'un éther ayant la formule [R-O]ₙ-R', dans laquelle R est un groupe alkyle substitué ou non substitué, R' est un groupe aryle substitué ou non substitué, et n est 1,2,3 ou 4 avec un agent d'acétylation choisi dans le groupe comprenant l'acide acétique et l'anhydrique acétique en présence d'au moins des quantités stoechiométriques d'un sel d'iodure de métal alcalin.

2. Méthode suivant la revendication 1, dans laquelle l'éther, l'agent d'acétylation et le sel d'iodure de métal alcalin sont présents dans des quantités environ stoechiométriques.

3. Méthode selon la revendication 1, dans laquelle la réaction est menée à une température comprise entre environ 130°C et environ 225°C.

4. Méthode selon la revendication 3, dans laquelle la réaction est conduite à une température comprise entre environ 160° C à environ 185° C.

5. Méthode selon la revendication 1, dans laquelle ledit éther comprend un éther d'aryle et de méthyle.

6. Méthode selon la revendication 1, dans laquelle ledit éther comprend un éther d'aryle et de diméthyle.

7. Méthode selon la revendication 1, dans laquelle le sel d'iodure de métal alcalin est choisi parmi le groupe comprenant le lithium et le sodium.

8. Méthode selon la revendication 7, dans laquelle le métal alcalin est le lithium.

9. Méthode selon la revendication 7, dans laquelle le métal alcalin est le sodium.
